(19)

**Europäisches Patentamt**
**European Patent Office**
**Office européen des brevets**

(11)  **EP 4 704 106 A1**

(12)  # EUROPEAN PATENT APPLICATION

(43) Date of publication:
04.03.2026 Bulletin 2026/10

(21) Application number: 24196876.7

(22) Date of filing: 28.08.2024

(51) International Patent Classification (IPC):
*G16H 40/20* (2018.01)

(52) Cooperative Patent Classification (CPC):
**G16H 40/20;** G16H 30/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **SCHMITT, Holger**
**Eindhoven (NL)**

• **SCHULZ, Heinrich**
**Eindhoven (NL)**
• **BERGTHOLDT, Martin**
**5656AG Eindhoven (NL)**
• **DESHPANDE, Hrishikesh Narayanrao**
**Eindhoven (NL)**
• **NETSCH, Thomas**
**Eindhoven (NL)**

(74) Representative: **Philips Intellectual Property &**
**Standards**
**High Tech Campus 52**
**5656 AG Eindhoven (NL)**

(54) **WORKLOAD-ADAPTIVE MEDICAL IMAGE GENERATION**

(57)  System (FS) for facilitating delivery of a medical data-based service (S), deliverable by cooperable resources (Rj). The system comprises an input interface (IN) for receiving a request for such a service at a predefined quality of service, QoS, the said QoS specifiable by a specification (Q) that specifies parameters. A load establisher (LE) establishes a current load of the resources (Rj). A QoS adjuster (QA) adjusts, based on the established load, one or more parameters, so that the QoS as per the other parameter is met.

Fig. 4

**Description**

FIELD OF THE INVENTION

The invention relates to a s system for facilitating delivery of a medical data-based service deliverable by cooperable resources, to a related method, to an arrangement, to a computer program element and to a computer readable medium.

BACKGROUND OF THE INVENTION

In today's clinical practice, in particular in radiology, when reconstruction of tomographic medial images is planned, parameters are typically taken into account that may affect the quality of the image itself (e.g. matrix size, reconstruction filter). The quality of the end-to-end diagnostic reporting workflow, in particular its turnaround time, however, is at present out of scope in this consideration. This means that, for example, the time to generate a report at a remote viewing station, given the current load of hospital network and reconstruction servers, are not at present considered when planning the reconstruction.
Specifically, reconstruction parameters today are defined statically, for example in the form of "exam cards" or other suitable data structure. For a given CT image acquisition, the matrix size of the image, slice thickness, slice spacing and other parameters affecting file size are pre-defined. After reconstruction, the images are typically transferred to a PACS or a viewing workstation via data communication network, where they are assessed by a radiologist, and a diagnostic report is generated.
Since the acceptable report turnaround time varies across different application scenarios (e.g. emergency situations vs. routine follow-up scans), and also the availability of resources to get the images ready and transferred to the radiologist varies over time (e.g. busy afternoon vs. midnight), it may be beneficial to the overall workflow to weigh at some or all these factors dynamically in order to optimize image reconstruction parameters.

SUMMARY OF THE INVENTION

There may therefore be a need for improved, in particular computing-based, service delivery arrangement, in particular in the medical field.
An object of the present invention is achieved by the subject matter of the independent claims where further embodiments are incorporated in the dependent claims. It should be noted that the following described aspect of the invention equally applies to the related method, the arrangement, to the computer program element and to the computer readable medium.
According to a first aspect of the invention there is provided a system for facilitating delivery of a medical data-based service, deliverable by cooperable resources (Rj), comprising:

an input interface for receiving a request for such a service at a pre-defined quality of service, QoS, the said QoS specifiable by a specification that specifies one or more parameters;
a load establisher capable of establishing a current load on at least one of the resources; and
a QoS adjuster capable of adjusting, based on the established load, one or more of the said one or more parameters, so that the QoS as per the other one or more parameter is met.

In embodiments, the said resources include any one or more of: a data communication network in which at least some of the said other resources are couplable, a medical imaging apparatus, a viewing station, an image generator capable of generating the imagery based on measurement data acquirable by the, or a medical imaging apparatus.
In embodiments, the image generator is any one or more of a: tomographic reconstruction engine, a data compression/decompression engine, a computer graphics rendering engine.
In embodiments, the adjustable one or more parameters pertain to operation of at least one of the resources.
In embodiments, the adjustable one or more parameters include imagery related parameters for operation of the image generator or of the imaging apparatus.
In embodiments, the system is capable of requesting/initiating image generation by the image generator at the adjusted one or more imagery-related parameters.
The requesting can be done automatically or upon user request via a user interface if user agrees to the proposed adjusted parameter. Instead of, or in addition to operating the image generator based on the adjusted parameter, operation of one or more of the other resources, such as of the imager/data measurement device, the renderer, etc.
In embodiments, the adjusting is based on a priority list of such parameters, indicative of an order in which such at least one parameter is to be adjustable for then being able to meet the QoS as per the at least one other parameter.

In embodiments, the adjusting may include adjusting an order of related requests in a pending queue at the one or more resources.

In embodiments, at least one of the at least one other parameter specifies an effective turnaround time for the delivery of the service.

In another aspect there is provided a computer-implemented method of facilitating delivery of a medical data-based service deliverable by cooperable resources, comprising:

receiving a request for such a service at a pre-defined quality of service, QoS, the said QoS specifiable by a specification that specifies one or more parameters;

establishing a current load on at least one of the resources; and

adjusting, based on the established load, one or more of the said one or more parameters, so that the QoS as per the other one or more parameter is met.

In another aspect there is provided an imagery-based service arrangement, comprising at least parts of the system as per any one of the preceding claims, and further comprising at least one of the said resources.

In another aspect there is provided a computer program element, which, when being executed by at least one processing unit, is adapted to cause the processing unit to perform the said method.

In another aspect there is provided at least one computer readable medium having stored thereon the program element.

The said resources can be all manners of devices, systems, machinery, such as computing or measurement devices, etc., preferably networkable. Some of the resources may be medical.

Examples envisaged herein are primarily based on (medical) image-based service, such as computed tomography, and certainly problems and issues solved are of particular importance in combination with tomographic modalities, such as modern spectral or photon counting CT scanners, etc., that can generate large amounts of high-resolution and/or multi-channel data. However, the proposed system may also be used in other imaging modalities.

The proposed system and method enable a clinical user to make an informed decision about image generation parameters, such as reconstruction parameters with respect to report turnaround time for example, such as is of interest in radiology-based services, or other.

A quality definition unit, or user herself, etc., may define and store in a memory MEM, database, etc., user preference QoS parameters, e.g., with respect to image quality and report turnaround time, or other. This can be done in the form of exam cards, or lookup tables, storing per type of exam the definition of ideal and acceptable image quality settings, as well as target turnaround time per exam type.

The load establisher, such as a resource monitoring unit, may be arranged as a computing module to monitor the availability and response time of workflow-relevant components such as image reconstruction hardware, hospital network connections and schedule of the future exams. Some of those components may be used up to a certain degree concurrently by other teams, users or devices, thus are shared as mainly envisaged.

The quality parameter adjuster may take the form of a reconstruction configuration unit/module. This module may take as input static data, such as the QoS specification of the quality definition unit or as specified by user, and the dynamic data as provided by the resource monitoring unit, and calculates as output one or more proposed image quality settings which is suited to fulfil in particular the report turnaround target under the current/instantaneous load (including availability) of resources. For present purposes, availability of a given resource should be construed as a special case of a load.

In some practical implementations, users may confirm the proposed parameter adjustment/setting. The QoS specification may specify at least two categories of parameters: those that can be adjusted (negotiable parameters) and those that cannot (non-negotiable parameters. In example of the latter is the effective (over all involved resources) report turnaround time.

Thus, as proposed herein in embodiments, availability, bandwidth, or other load aspects of a hospital resource(s) is/are taken into account herein for the end-to-end processing chain in effecting the delivery/providing of the requested service. In particular, the proposed system and method takes end-to-end turnaround time into account when calculating adjustments to the negotiable parameter(s).

The proposed setup may be of benefit in clinical settings, where the relative number of resources (resources per user) is limited, and resource sharing is needed.

*load /workload* pertains to instructions to deliver a task. The work(load) may be transactional, real time, database look up, analytical, etc. As used herein, availability, bandwidth, etc. are all example aspects of "load" on a resource as envisaged herein.

*(Quality of Service, QoS) parameter(s)* as used herein relate to, or are descriptive of, one or more various aspect(s) of the service, including performance, etc. The collection of some or all such QoS parameters forms a service specification. Preferably, one such QoS parameter may specify the overall turnaround time for completion/delivery of the service. The parameter may include multiple (sub-) parameters, Thus, reference to the singular "parameter" may not necessarily mean there is a single parameter. In general there are plural parameters. In particular, there may be two or more. One such parameter is said turnaround time, the other at least one may be an imaging/imagery/image-related parameter. The parameters that make up the service specification can be specified in whichever form or coding, such as "exam cards", LUTs, (associative) arrays, matrix, vector, tensor, or other data structure. It may include strings, numbers, a combination thereof, formulae, etc.

The service request may include implicitly or explicitly a request for a medical image to be generated, or for procurement of other data, such as lab data, etc. Service may be for the image as such, or for generation of radiology report based on such image. Service may be performed in part by human using applicable machinery, implement, equipment, apparatus, and the like, or may be itself automated such as diagnostic AI (artificial intelligence).

The QoS parameters may be in multiple categories: sacrificable/negotiable one(s) ("pawn" parameters), and non-negotiable (also referred to as "target") parameters: the earlier ones are free to be adjusted, whilst the latter ones ought to be maintained, heeded, adhered to. Thus, the target/ non-negotiable parameter(s) is/are maintained, at the expense of the negotiable parameter(s).

Another parameter category may appertain to the nature of such parameter, that is, what they relate to: some of the QoS parameters may be imagery/imaging related, whilst other such QoS parameters may be delivery related parameters. In general, the parameter may relate to quality of performance of respective ones of the resource. Other parameters may relate to other types of machinery equipment that is used in providing/delivering the service. The service is preferably medical image-based, such as a in a request for a radiology report, the image itself, or other, such as services around lab reports, assays, tests, radiation therapy, etc. The two (or more) said categories may overlap. Preferably, the negotiable/pawn parameters are imagery/imaging related ones.

*"user"* relates to a person, such as medical personnel or other, requesting the service. In other words, the user is in general not the patient, in respect of whom the service may be requested.

BRIEF DESCRIPTION OF THE DRAWINGS

Exemplary embodiments of the invention will now be described with reference to the following drawings, which, unless stated otherwise, are not to scale, wherein:

Fig. 1 shows a schematic block diagram of resources including a networked system of components capable of delivering a service;

Fig. 2 shows details of such a networked system capable of delivering a medical imagery-based service according to some embodiments;

Fig. 3 shows a timeline illustrating time expenses or overheads that together define an overall return time in respect of the service deliverable by a system of Fig. 1 or Fig. 2;

Fig. 4 shows a block diagram of a facilitator system operable to facilitate delivery of a service by using plural resources including a networked system of components capable of delivering such a service; and

Fig. 5 shows a computer implemented method of facilitating delivery of a service by using plural resources including a networked system of components capable of delivering such a service.

DETAILED DESCRIPTION OF EMBODIMENTS

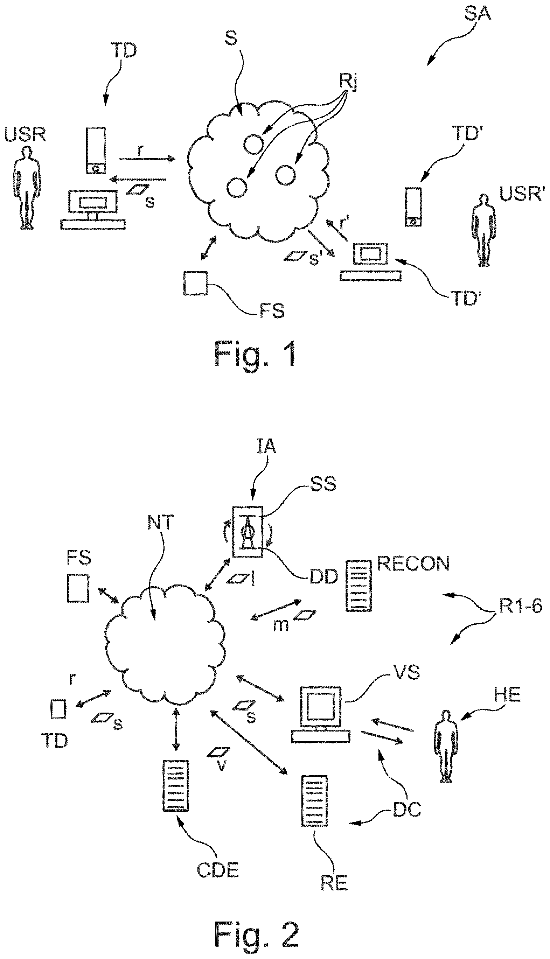

Reference is now made first to the block diagram of Fig. 1 which shows components of a medical data-based service delivery/provision arrangement SA.

The components of the service arrangement SA may comprise plural resources or components Rj (referred to herein from now on as "resources") that interact together to deliver service S to a user USR requesting same by putting through a corresponding request *r* via a terminal device TD. One such resource may be a data communication network NT, in which some or all other resources Rj are interconnectable in delivering the service. There may be plural such users USR' who each request such services via their terminal device TD'. Different users may request different such services, or some services may be similar, or even the same, among some users, as the case may be.

An example of such service S may be a radiology report *s,* or the delivery/provision thereof. Thus, for example USR may be a medical professional, such as a medical doctor, examining a patient, and it is felt that more examination is needed to come to a definite diagnostic conclusion. User USR may thus use the terminal device TD, such as a laptop, desktop, tablet, smartphone, etc., to put in an imaging request *r* through network NT, and for a related radiology report s that provides interpretational clues as ascertained by a radiologist or medical AI on interpreting the image. The request may be for the image as such, and the request of the report may then be implicit, or request as such may be for the report, in which case the imaging request is implicit, etc. For example, service request may explicitly or implicitly relate to an imaging request for patient's abdomen, chest etc., or any other, it being implicit that there is in addition such a radiology report needed, based on the requested image. Depending on the nature of the request for the service and context (such as time of day, etc.), there will be a (effective) turnaround time $T$ associated with the service request.

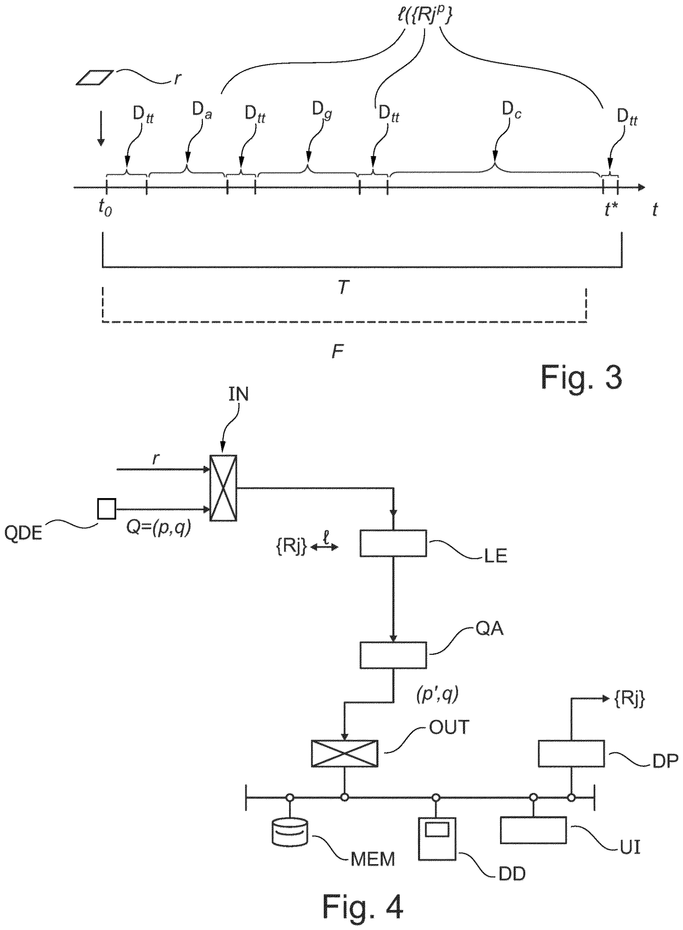

The turnaround time $T$ may be specified in a QoS specification as stored in a memory, such as in a PACS or HIS. The specification may be in terms of QoS parameters, $T$ being one such parameter, in particular a target parameter. The specification may have been previously stored by users themselves in their profile file for example, evoked on logon of user USR on their device TD. Alternatively, the QoS specification/parameters of which the specification is made up may be prescribed by a quality control/definition unit QDU (Fig. 4). The turnaround time $T$ is the time it takes between the request $r$ being put in by the user and when he or she receives the report, or requested service s is fulfilled. Thus, T is the effective time from service request coming until the service is fulfilled, delivered or provided for user USR to use.

As will be described below, some of the resources $R_j$ may be quite expensive to procure and run. Thus, the idea of procuring plural such devices/resources to be put at the disposal of multiple user USR, USR' may be potentially beyond reach for already cash-strapped national health care system, smaller medical outfits, etc. Certain medical establishments, such as smaller clinics, GP practices, or even large medial outfits in low-income environments may simply not be able to afford setting up the requisite number of high performance computing devices for example, or must make do with low performance devices, in an attempt to supporting a possibly large plurality of users USR, USR'. Thus, setting up shared such resources Rj may be the only feasible option in some cases.

What is proposed herein is the use of a facilitator system FS, a computerized arrangement, operable to facilitate the delivery of such service, in particular in the context of shared resources Rj, preferably interconnected in a network NT, the network being one of the resources Rj. The resources Rj may be subject to loads caused by various users and the processing of related data at or by the resources. There may be other limitations, such as latencies in the network, that all combine to result in an effective turnaround time $T$ for a radiology service s for example (they may be other examples) that is usually well in excess of the actual report turnaround time as pertains to the radiologist as such. That is, the effective turnaround time T may be well above the time it takes for the actual report being drawn up by the radiologist, or other human HE or AI expert, for example.

Thus, in more detail, what is proposed herein is to facilitate such service delivery by taking into account the effective overall turnaround time T for a given service, based on current load and capabilities of the resources Rj that interconnect and operate together to deliver the service. This allows a more realistic perspective on when an individual user USR, USR' may be able to expect the delivered service, such as the radiology report or any other medical data-based service, although (medical) imagery based services are primarily envisaged herein.

Broadly, the requested service S may be subject to certain of the said specified QoS parameters $Q$. The parameters $Q$ may reflect certain constraints or performance/quality parameters of the various resources Rj, operating together to deliver the service. Some of these parameters $p$ may be adjusted herein into adjusted parameters $p'$, to effect a lower quality in some aspects/parameters, but in such a way that other quality parameters $q$, deemed more important, are adhered to in the service S's delivery. For example, by lowering the quality of some of the operational quality parameters $p$->$p'$ of some of the resources involved, it can be assured that some other(s) of the parameter, the target parameters $q$, such as the overall effective turnaround time T, may be adhered to, as prescribed by the set of such parameters $Q=(p,q)$.

The facilitator system FS thus allows more efficient use of shared resources Rj by plural users USR, USR', possibly each sending in, at least at times, their various requests $r, r'$. Such efficient use of shared resources across plural users may be achieved by judicious adjustments of some of the respective quality parameters, referred to herein as negotiable ones $p$. Thus, as an example, some, or each user, or at least a majority of such users USR, USR' can still receive the, or their respective service S,S' in the requested turnaround time. The turnaround time may be considered a fixed target setting (non-negotiable), and can be achieved by bartering in some quality as per other parameters considered to be herein negotiable or "pawn" parameters $p$. The division of the set of QoS parameters $Q$ into negotiable $p$ and non-negotiable target parameters $q$ may be done by user in their profile, or may be prescribed by the quality definition unit QDU, as may be set centrally by the medical organization, the hospital, or locally by the respective departments, etc. The data structure in which $Q =(p,q)$ is stored, maintained or administered may support a field for the setting of related flags that are indicative as to whether the parameter to which the respective flag pertains is to be interpreted by the facilitator system FS as negotiable or not (target).

Reference is now made to Fig. 2, which shows a more detailed view of s set of such networked resources Rj. As an example, six such resources R1-R6 are shown, arranged in clockwise order. There may be more or less than six resources Rj, and their kind or nature may vary from what is shown in the example of the Figure.

Broadly, the resources Rj may be divided into infrastructural resources R1, such as the network NT itself, which is considered herein one of the resources, and operational or data consuming resources R2-R6, DC that process data, may produce intermediate data, which is then interchanged among the operational resources R2-R6 via the network R1, NT, to so together deliver the result S back to the requester USR's terminal device TD for example or storge in a memory MEM, such as PACS, etc. The terminal device TD may be a networked device with suitable network interfacing capabilities, thus is capable to interact with the network R1, NT and the remaining resources Rj. The same applies to the other users USR' on their terminal devices TD'. The terminal devices TD, TD' may or may not form part of the resources Rj.

In yet more detail, and with main reference to medical imagery-based service S delivery, at least one resource R2 is, or includes an imaging apparatus IA that is capable of acquiring measurement data $\lambda$ in respect of patient. A signal source SS of imaging apparatus IA may be used that emits an interrogating signal which interacts with imaged patient (not shown). The interrogating signals, upon such interaction with patient tissue, causes a response signal, which is then detected by the detector device DD. The response signal so detected forms the said measurements $\lambda$. The measurement data $\lambda$ may include recorded intensities, such as in transmission imaging. The detector is preferably capable of detecting the signal in digital form. The measurements $\lambda$ as recorded by the detector device DD may be provided as a two-dimensional grid/matrix of spatially resolved digital measurement values. The measurements $\lambda$ may be projection data, such as in X-ray based imaging, indeed envisaged herein in embodiments. Tomographic modalities, such as CT. MRI or other are examples of modalities envisaged herein. Such tomographic modalities are computationally expensive as they may require considerable computational overhead to turn the measurements into actual tomographic imagery. For example, the imaging apparatus IA, R2 may be an X-ray based imaging apparatus, such as projection-data only based radiography apparatus. However, in more advanced settings, the X-ray based imaging apparatus is of the said tomographic type, such as a diagnostic CT, an interventional C-arm, or any other. Rotational tomographic imaging apparatuses may be considered herein, but other tomographic modalities, not necessarily rotational, are also envisaged herein. The imaging apparatus IA, R2 can be of any modality, such as MRI, PET, Ultrasound (US), or any other. Alternatively, the measurement device is a digital microscope, such as may be used in digital histology/pathology.

Measurement data $\lambda$ so acquired can be sent through network NT to other one or more of the resources Rj, such as to an image generator equipment/machine IG, R3, that may run a high performance (dedicated) sever. Such image generator equipment IG, R3 may include a tomographic reconstructor machine/module RECON, IG, R3. Such a resource IG, R3 may handle the computationally, and potentially also memory-wise, demanding task of converting projection data, such as the received measurements $\lambda$, into a three-dimensional (cross-sectional ("slices")) image volume $m$ located in 3D space, for example, in the examination region between the signal source SS and the detector device DD, where the patient (in particular the region of interest ("ROI")) resides during acquisition. The tomographic reconstructor RECON, IG, R3 may be constituted by, or may be implemented on, one or more instances of high performance computing equipment, such as those that include multicore processor circuitry, or even more dedicated such processor circuitry, such as GPUs. Such high performance computing equipment may allow to run certain tomographic calculations more efficiently, such as via parallelization of the computational workload involved in some type of tomographic reconstruction algorithms. This may be useful in particular for tomographic reconstruction algorithms of the iterative type, where frequent re-calculations of back-projection operations and forward projection operations may be needed.

A negotiable parameter in respect of image generator may be a parameter in the rendering algorithm whose adjustment may cause less overhead at image generator, such as voxel density, slice thickness, certain filter kernel settings, number of iterations, etc.

Then, once the medical imagery $m$ is so generated by image generator IG, R3, it is forwarded through the network NT, R1 to a data consumer DC, such as viewing station VS, R4, on which the imagery $m$ may be visualized on a graphics display DD, using graphic interface circuitry or any other.

In some embodiments, a renderer RE,R5 may be used that converts the reconstructed (volumetric) imagery $m$ into computer graphics visual, such as 3D computer graphics ("CG"), which can then be visualized on the viewing station VS. Marching cubes, or any other rendering or CG algorithms may be used to accomplish this task. Again, running such CG algorithms may command quite high computational outlays, in particular computational equipment with potentially expensive GPU chipset. Thus, such CG equipment may again call for the mentioned high performance computing, and thus for associated costs. Thus, in settings where cash flow is a consideration, at least resources R3, R4 may need to be shared, potentially across a large number of clinical users USR, USR' which may create workload (referred to herein simply as "load") bottlenecks. A negotiable parameter in respect of renderer RE may be a parameter in the rendering algorithm whose adjustment may cause less overhead for that resource RE.

A human expert HE may view the imagery $m$ visualized on the display device DD of the viewing station VS, in a viewing session. The viewing session may take a certain amount of time which is part of the non-negotiable parameters mentioned above. At the conclusion of viewing session, human expert, draws up the requested report s, which can then be put into a PACS, or sent back through the network to the requesting device TD for the requester USR to consume. The report s may inform user USR, USR' decisions on diagnosis or therapy, etc. The viewing station VS may further run a word processor for example, used by user to draw up the report. Alternatively, or in addition, AI-based speech2text transcription tools or any other may be used.

Instead of, or in addition to, human expert HE, the image data $m$ may be consumed by a machine learning based radiology AI system, that processes imagery and automatically produces the report s. Whether by AI or human, the image data consumption for the actual drawing up of the report may come at a time cost, which may form one of the non-negotiable QoS parameters $q$.

In addition, but optionally to the processing by components R1-R5, some of the imagery may need to undergo compression/decompression, in order to achieve a manageable memory size. For example, some of modern CT imagers IA are capable of producing vast amount of image data, thanks to high resolution, thin slice thickness, etc. Thus, such a compression/decompression engine CDE, R6 may in itself form a resource R6 that needs to be used in order to send the data through the network and upon receiving it back is decompressed again to be so released for consumption by the requester USR, TD, or other resources R2-R5. Data compression and/or decompression may be based on compression/decompression algorithms, some lossy, others, preferably lossless. The compression/decompression calculation my cause load on the system R6, which may constitute a negotiable computational demand or QoS parameter, a constraint, which causes another time expense that may be considered if the service S is to be delivered within the overall set turnaround time $T$. For example, a compression/decompression parameter may represent the level of compression. Thus, a lower level of compression may ease workload, with less time expense spent on compression/decompression work, for example. The compression/decompression engine CDE may used not only for tomographic imaging, but also for radiography, such as in mammography or for handling of microscope imagery such as in digital histology.

Whilst the above is mainly concerned with medical imaging based resources, which includes at least the imager R2, IA or other measurement data acquisition resources, and/or the image generator R3, IG, optional CG renderer ER, R4, the principles described herein are not confined to such imaging settings. Any other or additional settings are also envisaged herein, such as for lab data, analytical results, blood tests, etc, that need to be coordinated, given a limited number of resources Rj versus a potentially large number of clinical or other users USR, USR'. Thus, the other resources, preferably networked in a wired or wireless or hybrid network NT, R1, may include, depending on the clinically relevant application, radiation therapy planning station, an assay system, mass spectrometer, or any other lab or non-lab equipment. Also, the resources R1-R6 in Fig. 2 are for illustration only. They have an exemplary character, but are nevertheless envisaged herein in embodiments, either singly, as any sub-selection, or as a whole. Other resources, in addition to or instead of the ones exemplified in Fig. 2, may be used. For example, another resource (not shown explicitly) may be a storage resource, such as PACS (Picture archiving and communication system), HIS (hospital information system), or other.

Fig. 3 illustrates a timeline $t$ for the overall turnaround time $T$, made up of time expenses $\Delta_j$ that are expended by the plural resources acting together, with the time expenses $\Delta_j$ adding up to provide the overall turnaround time $T$.

The overall effective turnaround time $T$, as mentioned earlier, is the overall time $T$ it takes from the service request r to come in at time point $t0$, and the time $t^*$ at which the service is delivered to the requester. This overall effective turnaround time is made up of one or more transfer times $\Delta tt$ that is consumed by the network NT, R1, an infrastructure resource, in exchanging data between the various other components $R_{j,j>1}$, such as resources R2-R6 as in the example of Fig. 2.

Thus, and referring back to the medical-imagery based embodiment as described above in Fig. 2, other time expenses $\Delta_j$ on top of transfer times $\Delta_{tr}$, may include any one or more of: the measurement data $\lambda$ acquisition time $\Delta_a$, that is the operation of the imaging apparatus, and the image generation time $\Delta_g$ consumed by, for example, by any one or more of rendering engine RE, the reconstructor engine RECON, the decompressor/compressor engine CDE, or any other of the resources that are operable on the measurement data, either directly, or indirectly on intermediate data derived therefrom. The image generation time $\Delta_g$ is the time(s) expended to produce the imagery $m$ consumable by image data consumer DC, or the time for transforming or reestablishing such image data, such as is the case for the compression/decompression overhead. Thus, there is then the data consumer time $\Delta_c$ which is essentially the time required by the human expert HE, such as radiologist, or AI, to consume the imagery $m$. For example, radiologist HE may review the imagery, and based on such review, writes up their report. Thus, user HE may spent time $\Delta_c$ operating the viewing station VS. Alternatively, time $\Delta_c$ is the time consumed by the diagnostic or radiology AI that is in charge producing the report, or assisting the human user HE in producing the report.

Thus, whilst the resources Rj are mainly operable automatically, this is not excluding involvement of human users, such as, e.g., the radiologist HE, or any other user who is operating or using the relevant resource/equipment, such as the viewing station VS, the rendering engine RE, or any other resource that is operable to deliver their part of the workload, on top of the workload previously done by the remaining resources upstream, in order to together, in concert, deliver the final result s.

Thus, the resources therein, whether human operated or automatically/ autonomously operating, are interconnected through the network NT, R1 to so facilitate data exchange or interchange, with some resources processing data as received from other resources, and so on. The interconnected resources Rj,j> 1 thus define a processing chain in which intermediate data is generated and exchanged between the components to deliver at one of the components, such as at the viewing station VS, the sought service/final result $s$. This processing chain may be linear in some but not all cases. Thus, the proceeding chain may also include loop(s), that is, some resources are revisited. The processing chain may be of hybrid type, thus, may include one or more loops and one or more linear sections, depending on the task at hand.

As indicated in the upper part of Fig. 3, the time expenses $\Delta_j$ are a function of the performance parameter $p$, as described by the quality services parameters $Q$, and the current load on the resources at the time the request r comes in. The overall turnaround time $T$ is then the sum $\Sigma_j \Delta_j$, or a function thereof, of some or all the time expenses involved in operation of respective resource Rj, each $\Delta_j$ being a function of performance parameters $p$ applicable at the time of the request, given the current load $\ell$ and the set of one or more non-negotiable parameter(s) $q$. The parameters $p$ may be adjusted to yield an actual effective (deliverable) overall turnaround time *F* that is less than or equal to prescribed effective turnaround time T. The said actual effective (deliverable) overall turnaround time *F is a* function of the current load, given the target parameter. F may be formulated as a function of the sum of the $\Delta_j$'s.

The QoS parameter *Q* may be stratified according to the service *S*. It and may be coded in structured or unstructured form, preferably the earlier, such as in an XML file, LUT, (associative) arrays, or any other. Each aspect of the service and the contribution of each contributing resource Rj are coded by a (performance) parameter that is either negotiable $p=(pj)$ or non-negotiable $q=(qj)$. It will be appreciated that in the above, reference to negotiable "parameter" *p* and non-negotiable "parameter" *q* of which the QoS specification *Q* is made up of, was done as a convenient shorthand. In reality one or both such parameter *p,q* may be any one or more of conceptualized, represented and stored as multi-dimensional data, in an *n*-dimensional or *m*-dimensional vector space $p = (p_1, ...p_j ...p_n)$, $q =(q_1, ...q_j ... q_m)$, wherein each parameter component $p_j$, $q_j$ appertains to the respective resource Rj. In general, some or each of the time expenses $\Delta_j$ are a function of pj or $q_j$. Some or each such component, may itself be made up of sub(-sub)-components, $p_j = (p_j^k)$, and similarly for the q's. Thus, the *p*'s and *q*'s may be represented as a respective vector or matrix, or even tensor. However, for convenience, in the following, there will be continued reference to "the parameter" *p* or *q*, unless specific reference to the sub(-sub) components is needed.

It is envisaged herein, that the facilitator system FS, whose block diagram is shown in Fig. 4 and to which reference is now made, is capable of negotiating the parameters *p*, to ensure that the non-negotiable parameters *q*, such as overall turnaround time T, can be maintained, given the current workload, by adjusting the negotiable parameter *p* to a setting *p'*. Such adjusted parameter *p'* will in general attract a lower quality of service in respect of the performance of one or more of the involved resources, but in general will still be capable to deliver an acceptable result for the clinical task at hand in the requested non-negotiable turnaround time *T*. Adjusting the negotiable parameter *p* into *p'* includes changing at least one of its components $p_j$ or $p_j^k$ .

Referring now in more detail to the block diagram in Fig. 4, this shows components of the facilitator system FS, operable as described above. The system includes an input interface IN through which is receivable the request *r* for service *S,* and explicitly or impoliticly, the applicable quality of service specification $Q=(p,q)$ as appertains to the requested service S.

A load establisher LE, at the time the request *r* is coming in, queries the collection of resources, in particular the network R1, NT, for its current load to establish the current load $\ell$. This load querying can be done by pinging the devices, in particular pinging the network by sending one or more small test/query data packets. The time of response may be an indication for the current load. Optionally, some or all of the other devices R2-R6 interconnected in the network R1, NT are so pinged individually, to so establish the respective load $\ell_j$ there. Any other form of load establishing, other than packet based pinging, may be used instead. Preferably, load establisher LE monitors the resources for their load at a suitable sampling interval (per 10s say), preferably quasi-constantly. The following metrics are examples of monitoring performance/load by the load establisher LE: -

(1) The current bandwidth (e.g. *MB/sec*) of the network NT, R (e.g., an ethernet connection) from the recon server R3, RECON or imager/scanner R2, IA (console) to one or more viewing workstations VS, R4, or any other connection between applicable resources;
(2) The current reconstruction performance (e.g. pixels/sec) of the recon server R3, RECON;
(3) The length of the reconstruction server queue.

Thus, as to metric (3), at some of the resources Rj, such as at the image generator IG, R3, or at the imager apparatus IA, R2, or other, there may already be a number of pending requests from the same USR or other users USR' for requested services in a queue. The length of the queue is preferably taken into account to establish the current workload. For example, the current workload may be multiplied by the length of the queue. Of note, the above load metrics at (1)-(3) are merely examples, whilst still envisaged herein in embodiments. Other(s), or additional, load monitoring metric(s) may be used.

A quality adjuster QA is operable to adjust the negotiable quality parameters *p* as per *Q,* based on the current workload and given the non- negotiable *q*, to calculate an adjusted parameter *p'*, which may then be output at an output interface OUT. Such outputting may include storing same on a memory, or displaying same on the displace device DD preferably at terminal device TD used by the user USR who requested the service S.

The user USR may then review the proposed adjusted parameter setting *p'* which, in particular, may attract a lower quality in respect one or more aspects, such as lower image quality ("IQ"), etc. This tradeoff between IQ vs adherence to non-negotiable parameter *q*, such as turnaround time T, can be considered by the user whether such is acceptable for him or her. If it is not acceptable, a new quality parameter adjustment may be requested by the user. In this case, if such a request signal is received, the load balance is re-established, which may have changed in the meantime, and the quality adjuster QA is re-operable to propose a newly adjusted quality parameter p", and so forth, until one is found to the user's satisfaction.

A non-reconciliatory message may be issued and displayed, which may state that no parameter setting *p'* can be found that can maintain the non-negotiable parameter(s) *q,* in particular the overall turnaround time T, given the current load. In this case the user may proceed in a different manner, may take other steps, as appropriate. If, however, a parameter adjustment *p'* is found, the system FS may be configured to allow user to approve such setting *p'*. For example, the system FS may support a user interface UI, such as graphical user interface, displayed on the display device DD or elsewhere. Once the user interacts with the user interface UI to approve p', a dispatcher DP is instructed to send the request through to the resources Rj concerned including the adjusted parameter setting p' as suggested by the quality parameter adjuster QA. The adjusted parameter settings *p'* are applied to the resources Rj concerned in delivering the service S. Alternatively, once a *p'* is found, this is automatically applied without seeking user approval first. A message may still be displayed to the user, or other indication may be provided, to inform user that service S was initiated and is being processed at the adjusted parameter *p'*.

For example, such an adjusted quality parameter setting p' may attract a lower image quality IQ. It may be put through to the imaging apparatus to change one or more of its settings for the given patient, for example, higher pitch, reduced scan range, or other parameters as pertain to operation of imager R2, IA, such as reduced angular sampling, or detector pixel binning.

In addition to, or instead of the above, certain parameter re-setting *p'* sacrifice on image quality at the image generator IG, R3 side, such as the reconstructor RECON. Thus, reconstructor IG, R3, RECON may be proposed to use a different parameter set for its reconstruction algorithm, such as any one or more of: increased voxel size/decreased voxel density, increased slice thickness, or any other, or a combination of the above, or other.

Thus, and in general, it is the task of parameter adjuster QA to adjust the reconstruction settings or other within the ranges defined in the quality specification Q, in an attempt to meet the target report turnaround time $q \ni T,$ under the current performance/load $\ell$ as monitored by the load establisher LE.

The following shows an example as to how the adjuster QA may work in order to sacrifice image quality for the purpose of maintaining the overall turnaround time T. The parameters in this case may be stored as fixed set parameters, as exam cards, or in other data structures. For example, based on user preferences, requirements affecting image quality are recorded and stored, for example:

(Table 1)

| Exam Type | Voxel Grid | Slice Thickness |
|---|---|---|
| CTA Brain ideal | 1536x1536 | 0.5 mm |
| CTA Brain fast | 512x512 | 2.0 mm |
| Cardiac CTA ideal | 1024x1024 | 0.4 mm |
| Cardiac CTA fast | 512x512 | 0.6 mm |
| Inner Ear ideal | 2048x2048 | 0.1 mm |
| Inner Ear fast | 1024x1024 | 0.3 mm |

In a similar way, target report turnaround time T is recorded and stored, for example as per the following table, such as exam card, or in any other data structure:-

(Table 2)

| Exam Type Report | Turnaround Time |
|---|---|
| CTA Brain Routine | 90 min |
| CTA Brain ER | 5 min |
| Inner Ear | 300 min |
| Cardiac CTA Screening | 60 min |
| Cardiac CTA ER | 5 min |

Example use scenario of load parameter adjuster QA:

A CTA Brain exam ought to be done for an ER (emergency) patient. The target report turnaround time *T* is prescribed at 5 min = 300 sec. The current network R1. NT bandwidth $\ell$ between the scanner R2, IA and the viewing workstation R4, VS as measured by the resource monitoring unit LE is 10 MB/sec. The ideal CTA Brain image settings would result in a total volume of 2700 MB, i.e. a network transfer time of 270 sec. Together with the time required by other components (recon server RECON R3 processing, processing of images on the viewing workstation R4, VS, etc.), this setting would violate the mandated 300 secs report turnaround time T. It is suggested by adjuster QA to the user USR that the voxel grid size be reduced *from p = 1536x1536* to *p' = 1024×1024,* resulting in a total volume of only 44% of the original value, which can be handled in the requested time under current circumstances. The user can either accept or dismiss the suggested modification by user interface UI.

In addition, or instead of the image quality-related parameter adjustments such as the reconstruction settings in above example, operation of parameter adjuster QA may resort to infrastructural re-settings in relation to some of the resources Rj. For example, image generator IG, R3, such as reconstructor, may maintain a priority waiting queue of scan raw data pending in a buffer of the reconstruction server RECON or other image generator IG, R3. The parameter adjuster QA may then cause to have raw data reconstructed in other than the chronological order, so to fulfil the report turnaround time T. Thus, in this embodiment, the negotiable parameter *p* may include the order of the pending reconstruction tasks in the queue, and such order may be adjusted into a different order *p'*. Optionally, facilitator FS may also output a reading worklist, including at which workstation VS to read which images, and in which order.

In general, operation of adjuster QA can be more generally framed as solving a system of inequalities $F(p) \leq T$ under constraints *C(pj),* given current load $\ell$:

$$F^{\ell}(p) = F(p_1, \ldots p_j, \ldots, p_n) < T \in q$$

(1a)

$$P_j \leq pj \leq P^j, j=1..n$$

(1b)

The $P^j$, $P_j$'s are required upper and lower bounds (numbers) to define intervals, within which the components of pj's of parameter p can be negotiated /adjusted. Objective function *F* is a real valued function on $\mathbb{R}^n$ and encapsulates the manner in which the performance and current load influences the effective overall turnaround time *T* over plural, such as all, resources $R_j$ involved in delivery of service S. For different services, the system *(1a,b)* may differ. Upper and/or lower bounds may be infinity oo. Function *F* may be linear or non-linear. Function *F* may be, or may be a function of, the sum of time expenses $\Delta_j$ of the resources Rj's involved in delivery of service S, such as mentioned above at Fig. 3.

Thus, objective function *F* is a parameterized function, parameterized by resource load $\ell$, and non-negotiable quality parameters *q* that are to be maintained, including the turnaround time *T,* and such function *F* is subject to a number of constraints *(1b)* that may include the negotiable parameters *p*. Changing some of the parameters *p* will in general come at a cost, such as reduced image quality, but on the other hand may still allow satisfying the objective function F as per *(1a).*

System *(1a,b)* may be solved by considering intersections of the, in general, high-dimensional hyper-half planes in $\mathbb{R}^n$. Numeric principles/algorithms from non-linear or linear programming may be used in more complex settings, to propose a suitable adjustment p*=p' to the negotiable parameters in order to meet the objective function *F.*

In some simple embodiments, a randomized approach may be used where in certain increments some of the negotiable parameter components $p_j$ (or more generally, $p_j^k$ ) are changed within the constraints *(1b),* either one by one or simultaneously, as needed, and every time the objective function is computed, optionally displayed. If F evaluates to less than or equal to *T,* the corresponding *p'* may be proposed to user as the suitable adjustment that satisfies in particular the return time *T.*

In a good number of cases, randomization within suitably small increments in one or more dimensions $p_j^k$, will yield a setting *p'* that satisfies (1a,b).

In some examples, such as the use case (tables 1,2) above, represent simple linear relationships such as between voxel size vs bandwidth that can be readily solved algebraically to yield an optional adjustment *p'*.

In another, more advanced embodiment, the above system of inequalities *(1a,b)* may be reformulated as a minimum or maximum optimization problem. For example, equations *(1a,b)* may be reformulated as a maximization problem.

$$p* = argmax_p \, F^\ell(p)$$

$$(2a)$$

$$P_j \leq \, pj \leq P^j, \, j=1..n$$

$$(2b)$$

$$F^\ell(p*)$$

If this maximum $F^\ell(p*)$ equals, or is less than, the prescribed turnaround time $T$, an arbitrary adjustment in any or more dimension $pj$ of $p*$ will then by definition yield a setting $p'$ that satisfy the constraints.

If the maximum $F^\ell(p*)$ is above the pre-set turnaround time $T$, some incremental changes according to one or plural dimensions of the optimal point p* may still yield a solution that respects the constraints. A randomizer may be used to sample the parameters to change some or plural parameter components in some or more dimensions of the optimal point $p*$. Thus, the optimal point $p*$ may serve as a "starting point" for finding valid parameter adjustments $p'$.

As a consistency test, the above at *(2a,b)* may be framed optionally as a minimization problem. If the minimum is above T, the non-reconciliatory message be issued. If not, only then is a solution of *(2a.b)* attempted.

In solving (2a,b), Lagrangian multiplier, or other gradient-based approaches, such as stochastic gradients, conjugate gradients, Newton, Nelder-Mead, etc. may be used.

In any of the above parameter adjustment setups, the incremental changes applied to $p*$ or at random for establishing $p'$ can be done arbitrarily. Thus, which one or more of the component parameters $p_j$, $p_j^k$ of $p$ or of $p*$ is actually adjusted, can be done by randomized selection. Thus, the performance of which resource Rj is to be changed (in general, for a lower quality) may be randomized. However, such randomization may not always be called for. Thus, as an alternative, the adjusting order is pre-determined, e.g., may be based instead on a priority list of the negotiable parameters $p_j$, $p_j^k$, indicative of an order $(i_1, i_2, ... . i_n)$ in which such the parameter components, if needed, are to be adjusted, to so meet the QoS as per one or more of the non-negotiable parameter $q$, such as $T$. Thus, first $p_{i1}$ is adjusted and it is checked whether $F$ drops below or equals $T$. If not, the next parameter $p_{i2}$ as per priority list $(i_1, i_2, ... . i_n)$ is adjusted, instead or on addition, and so. The priority list may be established by user USR, using user interface UI, or may be preset by the medical department, etc.

Instead of, or in addition to, the analytical approach as *eqs (1), (2),* machine leaning ("ML") may be used: in such setup, a training data collector system (not shown), including a logger or other, may be used for clinical users to collected data. Any adjustments that are being made in clinical practice are logged. In particular, such training data allows an ML model M to learn which sacrifices real world medical users are willing to make in clinical practice: the initial data, such as $\sigma = (p, q)$ and load $\mathcal{L}$, and the user adjusted ones $p'$ are stored in association to obtain the training data. Supervised, semi-supervised or unsupervised training algorithms can be used to train a suitable regression model, such as neural network, in a regression task to regress given $T$ and load into an acceptable adjusted parameter. Deep learning models with plural hidden layers between input and output layers may be used. The parameters may be interpreted as strings of characters and thus, a large language model (LLM), preferably of the transformer type with an attention mechanism, may be used. Other generative models, such as GANs, or other may be used.

It will be understood that the facilitator system FS as explained above may be operable in real time dynamic fashion. That is, once the request $r$ comes in, the proposed adjusted parameters $p'$ may be displayed, e.g. by appropriate dynamic GUI widgets in the user interface, such as bars that change in length, spinners or virtual hands of dial symbols that re-orient as the system repeatedly re-computes, over time, the applicable parameter changes that meet the pre-set target $T$ for the user to consider, given the respective load that may be expected to change over time. Thus, the user may choose to simply let the system re-compute at suitable sampling rates a sequence of new parameter adjustment suggestions $p'_{(k)}$, and the user can conveniently follow visually the changes on a "dashboard" as it were, e.g., made up of the said dynamic GUI widgets, until a suitable adjustment appears.

An accept button, or other as a GUI widget, may be actuated by the user through touch screen action, by pointer tool, or in any other way, to accept the system proposed parameter changes at a suitable time once found acceptable. A commit button may then be used to initiate/institute request via dispatcher, DP at those adjusted and accepted parameters, the service S. Thus, dispatcher DP causes the request to be sent through the network NT, and to the applicable resources, such as image generator or imaging apparatus, compression/decompression tool, CG renderer, as needed.

It will also be understood herein that the system FS is operable preferably in resource sharing among users USR, USR'. Thus, when, e.g., a given user USR uses the system FS, the load establisher LE and adjuster QA will take the pending requests or actual current resource usages of the other one or more users USR' into account when proposing parameter p' adjustments to the given user USR.

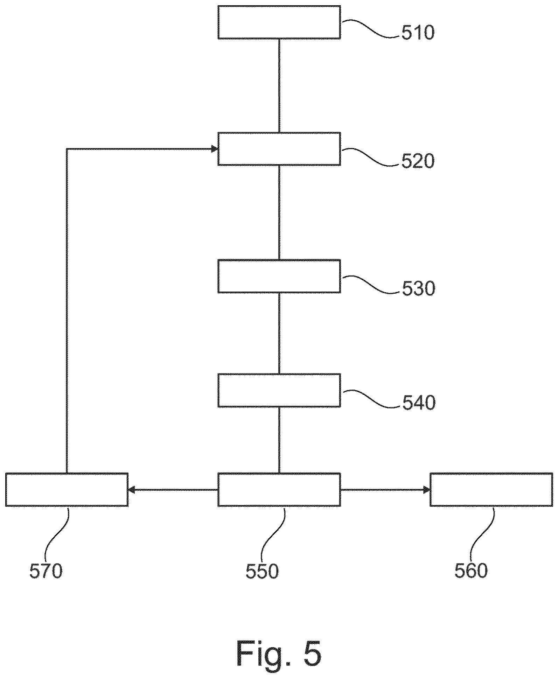

Reference is now made to Fig. 5 which shows a flow chart of a computer implemented method of facilitating delivery or service by co-operating computational nodes. Said nodes are entities (components, resources), connected in a data communication network, the network being one of the entities considered herein.

At step S510, a request for such a service deliverable by the interconnected components is received.

At step S520 a current load in the network and/or the interconnected components therein is established.

Based on this established load, at step S530 negotiable parts of a QoS parameter set $Q$ is adjusted to derive at an adjusted parameters p', which still allows applicable non-negotiable parameter settings $q$ of $Q$, such as the overall turnround time $T$, to be respected.

Such adjusted parameters may then be made available at step S540.

For example, the parameters may be displayed at step S550 on a display device to the requester USR.

At step S570 the proposed parameter may be rejected by user, and flow control returns to step S520 where the load is re-established, and a new parameter p" is proposed, and so forth, unless at step S560 the proposed adjusted parameter p' is accepted, at which point it may be committed for execution by the resources to deliver the service as requested.

The components of the system FS may be implemented as one or more software modules, run on one or more general-purpose processing units PU such as a workstation associated with the imager IA, or on a server computer associated with a group of imagers.

Alternatively, some or all components of the system FS may be arranged in hardware such as a suitably programmed microcontroller or microprocessor, such an FPGA (field-programmable-gate-array) or as a hardwired IC chip, an application specific integrated circuitry (ASIC), integrated into the imaging system IA In a further embodiment still, the system FS may be implemented in both, partly in software and partly in hardware.

The different components of the system FS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both a computer program that right from the beginning uses the invention and a computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. System (FS) for facilitating delivery of a medical data-based service (S), deliverable by cooperable resources (Rj), comprising:

   an input interface (IN) for receiving a request for such a service at a pre-defined quality of service, QoS, the said QoS specifiable by a specification ($Q$) that specifies one or more parameters;
   a load establisher (LE) capable of establishing a current load on at least one of the resources (Rj); and
   a QoS adjuster (QA) capable of adjusting, based on the established load, one or more of the said one or more parameters, so that the QoS as per the other one or more parameter is met.

2. System of claim 1, wherein said resources (Rj) include any one or more of: a data communication network (NT) in which at least some of the said other resources are couplable, a medical imaging apparatus (IA), a viewing station (VS), an image generator (IG) capable of generating the imagery ($m$) based on measurement data ($\lambda$) acquirable by the, or a, medical imaging apparatus (IA).

3. System of claim 2, wherein the image generator (IG) is any one or more of a:
   tomographic reconstruction engine (RECON), a data compression/decompression engine (CDE), a computer graphics rendering engine (RE).

4. System of any one of the preceding claims, wherein the adjustable one or more parameters pertain to operation of at least one of the resources (Rj).

5. System of claim 4, wherein the adjustable one or more parameters include imagery related parameters for operation of the image generator (IG) or of the imaging apparatus (IA).

6. System of any one of the preceding claims, capable of requesting image generation by the image generator (IG) at the adjusted one or more imagery-related parameters.

7. System of any one of the preceding claims, wherein the adjusting is based on a priority list of such parameters, indicative of an order in which such at least one parameter is to be adjustable for then being able to meet the QoS as per the at least one other parameter.

8. System of any one of the preceding claims, wherein the adjusting may include adjusting an order of related requests in a pending queue at the one or more resources.

9. System of any one of the preceding claims, wherein at least one of the at least one other parameter specifies a turnaround time ($T$) for the delivery of the service.

10. A computer-implemented method of facilitating delivery of a medical data-based service (S) deliverable by cooperable resources (Rj), comprising:

    receiving (S510) a request for such a service at a pre-defined quality of service, QoS, the said QoS specifiable by a specification (Q) that specifies one or more parameters;
    establishing (S520) a current load on at least one of the resources (Rj); and
    adjusting (S530), based on the established load, one or more of the said one or more parameters, so that the QoS as per the other one or more parameter is met.

11. An imagery-based service arrangement (SA), comprising at least parts of the system as per any one of the preceding claims, and further comprising at least one of the said resources (Rj).

12. A computer program element, which, when being executed by at least one processing unit, is adapted to cause the processing unit to perform the method as per claim 10.

13. At least one computer readable medium having stored thereon the program element of claim 12.

Fig. 1

Fig. 2

Fig. 3

Fig. 4

Fig. 5

Europäisches Patentamt
European Patent Office
Office européen des brevets

# EUROPEAN SEARCH REPORT

**Application Number**

EP 24 19 6876

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/138318 A1 (HAWKINS MICHAEL [US] ET AL) 28 May 2009 (2009-05-28)<br>* figures 1,3,5,6 *<br>* paragraphs [0027], [0031] *<br>* paragraphs [0051] - [0053] *<br>* paragraphs [0076] - [0079] *<br>* paragraphs [0083], [0084] *<br>----- | 1-13 | INV.<br>G16H40/20 |
| X | US 2008/155087 A1 (BLOUIN FRANCOIS [CA] ET AL) 26 June 2008 (2008-06-26)<br>* figure 4 *<br>* paragraphs [0026] - [0052] *<br>----- | 1-13 | |
| | | | **TECHNICAL FIELDS SEARCHED (IPC)**<br><br>G16H |

The present search report has been drawn up for all claims

| Place of search | Date of completion of the search | Examiner |
|---|---|---|
| Munich | 28 January 2025 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 6876

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2025

| Patent document cited in search report | Publication date | Patent family member(s) | Publication date |
|---|---|---|---|
| US 2009138318 A1 | 28-05-2009 | NONE | |
| US 2008155087 A1 | 26-06-2008 | NONE | |

EPO FORM P0459

For more details about this annex : see Official Journal of the European Patent Office, No. 12/82